# EUROPEAN PATENT APPLICATION

(11) **EP 4 029 440 A1**
(43) Date of publication of application: **20.07.2022**
(21) Application number: 21151759.4
(22) Date of filing: 15.01.2021
(51) Int. Cl.: A61B 5/055, A61B 5/00, A61B 5/0205

(54) **MRI WAKEFULNESS MONITORING**

(71) Applicant: Koninklijke Philips N.V., 5656 AG Eindhoven (NL)
(72) Inventor: VAN EE, Raymond, 5656 AE Eindhoven (NL); WESTERINK, Joanne Henriëtte Desirée Monique, 5656 AE Eindhoven (NL); LEUFKENS, Timmy Robertus Maria, 5656 AE Eindhoven (NL); HUIJBERS, Willem, 5656 AE Eindhoven (NL); MENA BENITO, Maria Estrella, 5656 AE Eindhoven (NL); DENISSEN, Adrianus Johannes Maria, 5656 AE Eindhoven (NL)
(74) Representative: Philips Intellectual Property & Standards

(57) **Abstract**

The present invention relates to a method (10) for monitoring the wakefulness of an imaged subject during an MRI examination. The method comprises acquiring (11) at least one magnetic resonance image of the subject using an MRI system (100), and determining (13) a wakefulness measure based on at least a part (I) of the acquired image or images and/or on EEG data obtained from the imaged subject during the acquisition. The method also comprises generating (14) at least one signal if the determined wakefulness measure indicates that the subject has fallen asleep, has woken up and/or is predicted to transition from an wake to a sleep state and/or vice versa. The signal is generated during the acquisition (11) and/or before removing the subject from the MRI system. Further aspects of the invention relate to computer program products, computer systems and MRI systems.

## Description

### FIELD OF THE INVENTION

The present invention relates to the field of magnetic resonance imaging, and, more specifically, to a device and method for monitoring a state of wakefulness of a subject during a magnetic resonance imaging procedure, and to a related magnetic resonance imaging system and computer program product.

### BACKGROUND OF THE INVENTION

Magnetic resonance imaging (MRI) allows internal body structures in the human or animal body to be imaged non-invasively. It is particularly useful for studying the brain, since a wide variety of different MRI techniques are known that allow different properties and/or structures of the brain to be detected and visualized. For example, functional magnetic resonance imaging (fMRI) techniques are known that can detect subtle contrast changes indicative of blood oxygenation, such that it can be determined which areas in the brain are active and/or inactive, and, more specifically, such that local changes in brain activity over time can be detected. This is a versatile approach to investigate the functioning of the human brain, e.g. for research purposes, as well as for diagnosing neurological, psychiatric or other diseases and disorders involving the brain. Specific sensory, cognitive, emotional, behavioral, motivational, memory and motor functions of the brain can be visualized and studied in detail using fMRI. Thus, the involvement in specific actions of the brain (but not necessarily limited to direct and/or active tasks) of specific regions in the neocortex and the limbic system can be studied in detail.

Of course, many other useful MRI techniques exist that can provide useful anatomical, physiological and/or functional information regarding the examined brain, such as cerebral perfusion MRI, e.g. using arterial spin labeling or tracing of a contrast agent bolus, and/or diffusion MRI, e.g. which (amongst other applications) enables white matter tractography.

Unfortunately, during an MRI examination, a subject can fall asleep. This can even occur rather frequently, particularly in cases where patients are suffering from conditions that can adversely affect wakefulness or reduce the capacity to focus and stay alert. Those conditions may include a wide range of possible illnesses, traumas and disorders, and can even be as simple as age-related effects, e.g. a tendency to fall asleep for old as well as very young subjects. A condition that heightens the risk of falling asleep during the procedure could even be the underlying reason for performing the MRI examination, or could be related to a suspected disease or disorder for which a diagnosis is sought. For example, patients suffering from a psychiatric illness, e.g. particularly in case of a major depressive disorder, tend to have a higher likelihood of experiencing sleep issues, which can cause higher levels of sleepiness during the day. Also, a number of psychoactive pharmaceuticals, e.g. tricyclic antidepressants, benzodiazepines and anti-psychotics, have sedative properties, which can also contribute to the chance of falling asleep while being imaged.

Factors that are related to the scanner and the MRI imaging procedure, can also contribute to the likelihood of falling asleep. Many MRI protocols can take quite some time, such that the patient has ample time to fall asleep. Even though an MRI scanner, in operation, can make loud sounds, this does not necessarily contribute to the patient staying awake. For example, to the contrary, rhythmic noises (e.g. which may be caused by rapidly switching field gradients) can have a calming, almost hypnotic, effect. Patients are also often provided with earmuffs, due to the loud scanner sounds, which can lead to the patient feeling detached from a normal sensory experience. Other factors include that the patient is typically lying relatively comfortably inside the MRI bore, and asked to refrain from movement.

When a patient (or other subject) falls asleep during the recording of an MRI brain scan, the activity of the brain will clearly change dramatically. It is well-known that brain activity in a sleeping state is quite different from wakeful activity. When the purpose of the examination is to perform a functional evaluation of the brain (or specific brain regions), this is clearly detrimental to the quality of the collected data, possibly to the extent of rendering the data entirely useless. For example, this may influence a proper diagnosis or analysis of fMRI data, and may even decrease the test-retest reliability of resting-state fMRI.

An increasing usage of autonomous brain scanning is foreseen for diagnostic and psychiatric purposes, e.g. as a source of information to take into account in diagnosis and treatment planning of psychiatric patients. If a patient falls asleep during such a brain scanning operation, this can severely confound the gathered information and, consequently, the diagnosis and/or treatment plan. In autonomous brain scanning, a resting-state scan is acquired, in which the patient does not engage in any sensory or cognitive task (e.g. contrary to conventional task-related fMRI paradigms). Such resting-state scan can clearly be quite boring to the patient, particularly if the scan procedure takes longer than (e.g.) 25 minutes. Moreover, the resting-state scan can be performed with closed eyes, which also does not contribute to the patient staying awake.

Also in a more conventional task-related fMRI paradigm, the patient may receive a boring visual stimulus and/or may be instructed to perform a boring, repetitive task. For such tasks, the patient's attention can gradually shift away, resulting in brain scans that do not contain the intended information. This can lead to inconclusive results, or even worse, to incorrect diagnoses.

While a patient who falls asleep can be clearly problematic in the case of functional MRI, this problem is not limited to this area of application alone. Many physiological parameters change substantially when a patient is asleep and/or transitions to a state of drowsiness and sleep onset. To name a few examples, those physiological parameters include respiratory features, such as the breathing rate, pattern and depth, circulatory system features (e.g. the heart rate, electrocardiogram and the blood pressure), the body temperature, and various blood concentration levels, e.g. of oxygen, carbon dioxide and glucose. Any MRI examination that involves, or could be substantially affected by, such a parameter may fail, or the results thereof could be less reliable or representative of the conditions intended to be tested.

It is noted that such problems are also not necessarily limited to MRI studies of the brain either, even though brain imaging remains an area of application where a lack of attention of the patient and/or the onset of sleep during the imaging procedure might be considered to be, presumptively, in many cases a serious issue. For example, a drowsy or sleeping state of the patient can also cause involuntary movements, which in their own right can be disruptive to the image acquisition or detrimental to the data quality, regardless of the type of MRI protocol being applied.

When an examination aims at analyzing respiratory function, it is to be noted that respiration synchronized image acquisition may rely on an accurate tracking of the respiration waveform, which in many cases may presume a periodic and/or reproducible breathing pattern. However, when the subject falls asleep, this waveform may change substantially, e.g. the amplitude decreases and the period may lengthen, such that the algorithm used to track the respiratory pattern may fail or becomes less accurate. For a respiratory evaluation by MRI imaging, a normal (daytime) breathing pattern may be needed anyhow for the intended purpose, even if a respiratory monitoring algorithm can compensate for a change of the state of the patient. Even if the study is not (primarily) concerned with respiratory function as such, the imaging protocol may still rely on respiratory tracking for the purpose of motion compensation, e.g. using a respiratory gating technique.

Furthermore, a patient who startles awake, particularly in an unfamiliar environment such as a scanner bore and/or with his/her head in a head coil assembly, could experience distress, and could even react in a way that might damage equipment or that poses a risk to the patient's safety.

Therefore, a need exists in the art for methods and means to detect when a patient is sleeping, falls asleep or transitions through a phase of increasing drowsiness during an MRI examination, and to take action in response to such detected event, e.g. alerting an operator and/or providing a feedback to the patient to increase his/her alertness.

The United States patent application no. US 2014/055133 discloses an MRI system that is adapted to dynamically adjust the color and brightness of lighting, which a patient experiences inside the scanner environment, by controlling illuminators mounted on the inside of the MRI bore accordingly. The eye pupils of the patient are detected by camera image processing, and the illumination is adjusted when the dilation of the pupils has changed substantially with respect to a reference size that was determined earlier. Thus, the lighting can be used to awaken the patient, e.g. by increasing the light intensity and changing the color to a color that is considered to promote alertness.

### SUMMARY OF THE INVENTION

It is an object of embodiments of the present invention to provide in good and efficient methods and means to monitor the wakefulness of an imaged subject during a magnetic resonance imaging (MRI) procedure, e.g. to detect and predict when a patient is falling asleep during an MRI examination.

It is an advantage of embodiments of the present invention that a sleep/awake state of the imaged patient can be monitored and/or predicted during an MRI examination of the brain, such that information obtained by the brain scan is representative of the correct state of the patient, i.e. the acquired images represent the intended information, such that wasted resources can be avoided, e.g. when a scan could not lead to a conclusive analysis due to a patient changing sleep/awake state unexpectedly during the examination, and/or such that an inappropriate, e.g. incorrect, diagnosis on the basis of incorrect assumptions regarding the state of the patient during the image acquisition can be avoided.

It is an advantage of embodiments of the present invention that a sleep/awake state of the imaged subject can be monitored and/or predicted, e.g. in substantially real-time, during functional magnetic resonance imaging (fMRI) examination, such that poor task performance due to the patient falling asleep can be avoided. As already mentioned hereinabove, the quality of resting-state fMRI information can also suffer from a patient falling asleep (when intended to stay awake), which problem can likewise be advantageously avoided or reduced by embodiments of the present invention.

It is an advantage of embodiments of the present invention that detecting and/or predicting (e.g. and preventing upon prediction) a sleeping state of a patient can improve image quality and/or operational efficiency in many MRI examinations, including but not limited to studies of the brain, the lungs, the heart, etc. While in most cases the patient will be studied while being awake, and is presumed to be in that state while being imaged, embodiments of the present invention can also advantageously detect and/or predict a patient waking up when the opposite assumption is made, e.g. when explicitly studying properties of the patient's sleep pattern or when a patient is imaged under sedation.

It is an advantage of embodiments of the present invention that, in response to the detection of a patient falling asleep and/or detecting an increasing drowsiness and predicting the onset of sleep, the patient may be prevented from falling asleep or may be woken up by emitting a feedback signal, such as a movement by an actuator.

It is an advantage of embodiments of the present invention that brain networks and changes in brain activity due to changes in wakefulness, e.g. due to a patient falling asleep during a scan, are not confused with brain networks and neural activity that reflect a psychiatric illness, such that the risk of an incorrect diagnosis of a psychiatric patient based on MRI imaging is avoided or reduced.

It is an advantage of embodiments of the present invention that an automatic approach is provided to detect, and prevent, the onset of sleep during MRI scanning.

It is an advantage of embodiments of the present invention that a predetermined time of scanning, e.g. a predetermined number of images spaced over a sufficient time, is ensured during which the imaged subject is awake. For example, it is an advantage that the number of collected MRI images and/or the total time over which images are acquired can be automatically increased to compensate for time segments during which the imaged subject was detected to be asleep or drowsy.

It is an advantage of embodiments of the present invention that different actions can be automatically taken, e.g. of different intensity, to prevent a patient from falling asleep and to wake up a patient that is asleep.

It is an advantage of embodiments of the present invention that MRI images, e.g. a fMRI timeseries, can be annotated with information regarding a state of wakefulness, drowsiness and/or sleep of a patient, such that images acquired while the patient was asleep or falling asleep can be processed differently (e.g. discarded, disregarded, given lesser weight, ...) than those in which the patient was fully awake. For example, such annotation information can also be taken into account in the analysis of fMRI timeseries data.

A device and method in accordance with embodiments of the present invention achieves the above objective.

In a first aspect, the present invention relates to a method for monitoring the wakefulness of an imaged subject during a magnetic resonance imaging examination. The method comprises acquiring at least one magnetic resonance image of the subject using a magnetic resonance imaging system. The method comprises determining a wakefulness measure based on at least a part of the acquired image or images and/or on electroencephalography data obtained from the imaged subject during said acquisition. The method comprises generating at least one signal if the determined wakefulness measure indicates that the subject has fallen asleep, has woken up and/or is predicted to transition from a wake to a sleep state and/or vice versa. The signal is generated during the acquisition and/or before removing the subject from the magnetic resonance imaging system.

In a method in accordance with embodiments of the present invention, the determined wakefulness measure may comprise or consist of a prediction of the subject falling asleep within a predetermined period of time.

In a method in accordance with embodiments of the present invention, the at least one magnetic resonance image may comprise a timeseries of magnetic resonance images, and the wakefulness measure may be determined when some, but not all, of the images in the timeseries have been acquired.

In a method in accordance with embodiments of the present invention, the wakefulness measure may be determined by taking brain activity into account in at least one predetermined region of interest of the brain that is involved in the management of sleeping and/or waking states of the brain. The brain activity may be detected by a blood oxygenation level dependent effect in the region of interest in said part of the acquired image or images.

In a method in accordance with embodiments of the present invention, the at least one predetermined region of interest may comprise or consist of the ventrolateral preoptic nucleus and/or the median preoptic nucleus of the hypothalamus.

In a method in accordance with embodiments of the present invention, the wakefulness measure may be determined by taking at least one cardiorespiratory parameter into account that is determined based on said part of the acquired image or images.

In a method in accordance with embodiments of the present invention, acquiring the at least one magnetic resonance scan (involving multiple images) may comprise acquiring a primary image or images, in which the acquisition switches from acquiring a primary image or images of the subject for a diagnostic or other medical purpose to acquiring a secondary image or images forming said part for determining the wakefulness measure and back to acquiring said primary image or images.

In a method in accordance with embodiments of the present invention, determining of the wakefulness measure may take further sensor data into account that are collected during the acquisition of the image or images. The further sensor data may comprise an electrocardiogram, a photoplethysmogram, a cardioballistogram, an electromyogram, an optical or infrared camera image or video, and/or radar or lidar data.

In a method in accordance with embodiments of the present invention, generating the signal may comprise generating an alert for an operator of the magnetic resonance imaging system to inform the operator that the subject has fallen asleep or is predicted to fall asleep.

In a method in accordance with embodiments of the present invention, generating the signal may comprise an instruction to the magnetic resonance imaging system to extend a time series of images being acquired, to repeat the acquisition of the image or images or part thereof, to terminate the acquisition and/or to pause the acquisition.

In a method in accordance with embodiments of the present invention, the generated signal may comprise a sensory stimulus cuing the subject to wake the subject up and/or to reduce the risk of the subject falling asleep.

In a method in accordance with embodiments of the present invention, the sensory stimulus may be delivered in a stepwise or continuously varying manner so as to draw the attention of the subject without startling the subject.

In a method in accordance with embodiments of the present invention, the generated signal may comprise annotation information for storing along with the acquired magnetic resonance images, such that an evolution of the wakefulness measure during the acquisition and/or a timing of cuing the subject with said sensory stimulus(/i) can be taken into account when analyzing said magnetic resonance images.

In a second aspect, the present invention relates to a computer program product comprising machine executable instructions for execution by a processor, wherein execution of the machine executable instructions causes the processor to perform a method in accordance with embodiments of the first aspect of the present invention.

In a third aspect, the present invention relates to a computer system for monitoring the wakefulness of an imaged subject during a magnetic resonance imaging examination. The computer system comprises:
- an input for receiving, in substantially real-time, at least one magnetic resonance image of the subject acquired using a magnetic resonance imaging system and/or electroencephalography data obtained from the imaged subject during said magnetic resonance imaging,
- an output, and
- a processor.

The processor is adapted for determining a wakefulness measure based on at least a part of the acquired image or images and/or based on the electroencephalography data. The processor is also adapted to generate at least one signal via the output if the determined wakefulness measure indicates that the subject has fallen asleep, has woken up and/or is predicted to transition from a wake to a sleep state and/or vice versa. The processor is adapted to generate the signal during said image acquisition, e.g. while a stream of image or EEG data received via the input has not yet terminated.

In a fourth aspect, the present invention relates to a magnetic resonance imaging system comprising a computer system in accordance with embodiments of the third aspect of the present invention.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a magnetic imaging system in accordance with embodiments of the present invention.
Fig. 2 shows an illustrative method in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

Where reference is made to "real-time" or "near real-time" in the present disclosure, it will be understood by the person skilled in the art that processing operations necessarily require at least some time for computation, such that the real-time or instantaneous processing is obviously constrained by technical capabilities. However, the use of "real-time" indicates that the processing being performed is time-critical in nature, and preferably performed swiftly to allow actions to be taken in response to the determined result, while this result is still relevant. Particularly, in embodiments of the present invention, a wakefulness state of an imaged subject, e.g. a patient, is determined during the acquisition of MRI images, such that actions can be taken in response to the determined state while that image acquisition (i.e. the acquisition of a sequence of images) has not yet been completely terminated. Therefore, the processing to determine that state and to determine a suitable response thereto is performed (substantially) instantaneously, or at least quickly such as to approximate an instantaneous action. For example, this allows a corrective action to be taken while continuing to acquire MRI images. Therefore, a (near) real-time may refer to a processing time of (only as an example) less than two minutes, or less than one minute, preferably less than 30 seconds, or, even more preferred, less than 10 seconds. Clearly, if a processing time of even substantially less than that, e.g. less than one second, can be achieved without excessive impact on costs (e.g. costs of the required computing hardware, operating power etc.), this may be even more preferred. However, at least some of the embodiments of the present invention may involve alerting an operator of a result based on the (near) real-time computations, who can then take an action in response. This may imply that, considering the time required for the operator to take a responsive action when necessary, the added value of reducing the processing time to less than (for example) a few seconds could be somewhat limited (e.g. unless an automated response is provided as alternative or in addition to alerting the operator).

In a first aspect, the present invention relates to a method for monitoring the wakefulness of an imaged subject during a magnetic resonance imaging examination. Particularly, the method in accordance with embodiments of the present invention may be a computer-implemented method, e.g. performed by a computer program product when executed on a suitable computing platform (however, embodiments that are completely or partially implemented in computing hardware, e.g. digital processing hardware, are not excluded). The method may be applied to determine when a patient is falling asleep, and to prevent this from happening and/or restore wakefulness of the patient, during a magnetic resonance imaging examination.

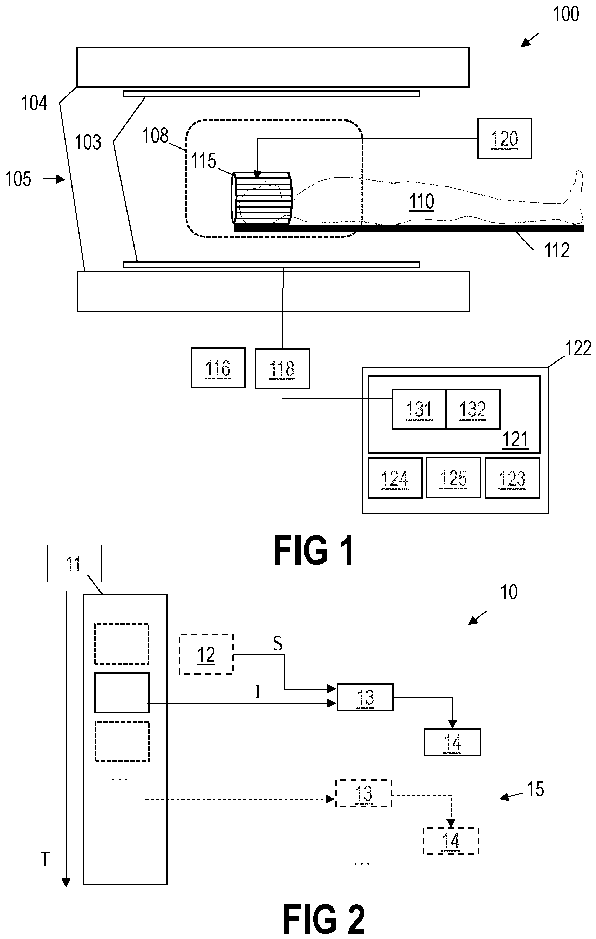

The method may be performed by interacting with a magnetic resonance imaging system. Fig. 1 shows an example of such magnetic resonance imaging system 100, e.g. an MRI system that is generally suited to implement step of a method in accordance with embodiments of the present invention. The MRI system 100 comprises a magnet 104, e.g. a superconducting cylindrical magnet through which a bore 105 is defined. Variations of and/or alternatives for this type of magnet are also possible, e.g. the magnet may be a split cylindrical magnet or an open magnet (e.g. in which magnet sections are arranged around, e.g. above and below, an open space for receiving the subject to be imaged). The magnet 104 may comprise a plurality of superconducting coils, e.g. in a cryostat. Inside the bore 105, an imaging region 108 is defined in which a subject can be imaged. In this imaging region 108, the magnetic field of the magnet 104 is typically strong and uniform, i.e. sufficiently strong and uniform to perform MR imaging. For an open magnet or other alternative magnet assembly, there might not be a bore cavity defined, but the imaging region 108 will still be clearly identifiable, e.g. as characterized by a suitable magnetic field strength and homogeneity. The magnetic resonance data acquired by the system, in use thereof, is typically representative of the content of the space defined by the imaging region 108, or a part thereof, e.g. a specific region of interest defined inside this imaging region.

A subject 110, e.g. a human subject such as a patient to be diagnosed or examined, can be, in use, placed inside the imaging region 108. For this, the system may comprise a subject support 112, e.g. an actuated patient couch. By controlling the subject support, a body part or region of interest in the subject on the support can be moved to a suitable position inside the imaging region 108.

The system may further comprise a plurality of magnetic field gradient coils 103, adapted to spatially encode magnetic spins within the imaging region 108 (e.g. by superimposing local magnetic field variations onto the strong magnetic field of the magnet). For example, the magnetic field gradient coils may comprise at least three coils (or sets of coils) to allow spatial encoding in three orthogonal spatial directions, i.e. in any direction in space. The magnetic field gradient coils 103 may be controlled as a function of time, in addition to controlled such as to select the spatial encoding direction, e.g. to create gradients that are pulsed, ramped or otherwise varying as function of time. For example, a power supply (or power supplies) of the magnetic field gradient coils may be controlled by a controller, processor or computer to achieve a magnetic field (gradient) in accordance with a desired spatial and/or temporal function. The system may typically comprise a controller 118 to control the magnetic field gradient coils 103.

The system may typically comprise a radiofrequency (RF) coil 115 (an RF antenna device) which may act as a radiofrequency receiver and transmitter. For example, the RF coil may transmit a signal that disrupts the alignment of (nuclear) spins with the magnetic field (of atoms and molecules in the imaging region 108), and the RF coil may receive a return signal that is generated by a precession and relaxation of the disrupted nuclear spins back to the magnetic field alignment. The RF coil may be a construct comprising a plurality of separate coil elements, and/or support equipment. The RF coil may be integrated in the bore system, or may be a separate component to be placed close to or in contact with the imaged subject. For example, the RF coil(s) may be integrated in a head coil assembly for imaging the brain of the subject 110.

The RF coil 115 comprises, or is connected to, an RF transceiver 116. Obviously, the system may also comprise separate RF transmission and receival antennas and corresponding (separate) receiver and transmitter systems, or any combination of a plurality of antenna coils and transceiver and/or receiver and/or transmitter systems. For example, a plurality of antenna coils may be connected to separate receive and/or transmit channels, which may be controlled separately, in unison or in any combination that is considered suitable for the intended purpose. A number of parallel channels may, for example, be useful for parallel imaging techniques, such as SENSE or ASSET (sensitivity encoding; array coil spatial sensitivity encoding).

The MRI system 100 may also (optionally) comprise a sensory stimulus source 120 to provide a sensory stimulus, an entertainment feed and/or information to the subject during an MRI imaging operation. Such sensory stimuli may include a signal that can be provided to the subject via any of (or combination of) the physical senses of the body, such as touch, smell, taste, hearing and sight. Commonly, the sensory stimulus source may include a light or display to present a visual stimulus to the subject while positioned in the imaging region (including possibly a projection screen and/or optical system to relay displayed information to within the bore volume), and/or a speaker or sound source to present an auditory stimulus to the subject (including possibly a relay system, e.g. using guided air pressure transmission or contact conduction of sound into the bore volume, e.g. to a headphone).

The sensory stimulus source 120 can be used to provide entertainment to the patient, to inform the patient about the progress of the imaging procedure being performed and/or to intentionally trigger activity in a brain region of interest in the subject (e.g. in a functional MRI examination). The sensory stimulus source 120 may also be used, in accordance with embodiments of the present invention, to provide a stimulus to the patient to restore or promote wakefulness, e.g. in response to a detected or predicted onset of sleep. Such use to improve wakefulness will be discussed in more detail further hereinbelow.

The RF transceiver 116, the gradient coil controller 118 and (optionally) the sensory stimulus source 120 may be connected to a hardware interface 121 of a computer system 122. The hardware interface may, for example, be a digital network interface or data bus interface. The hardware interface may be adapted for exchanging data and/or commands between the computer system and other components of the system. The hardware interface may also be adapted for exchanging data and/or commands between different components of the computer system. The hardware interface is not necessarily a homogenous interface infrastructure, e.g. may comprise or consist of different types of data exchange media, computer networks, communication buses, shared memory access structures and the like.

The MRI system 100 is adapted for acquiring MRI images. This may also comprise, optionally, the acquisition of fMRI images, e.g. the system may be adapted to perform additional processing and/or timing control specifically for fMRI imaging, e.g. to acquire and/or analyze fMRI data. Thus, images can be acquired and reconstructed, e.g. by the computer system 122, based on signals detected by the RF transceiver and the antenna coil(s) connected thereto. For brain imaging, e.g. fMRI (without limitation thereto), the RF coil(s) may be integrated in (or comprise) a head coil assembly to achieve a good signal quality for imaging a subject's brain.

Embodiments in which the computer system comprises a plurality of computers, interconnected by a suitable network infrastructure are not necessarily excluded. For example, processing performed by the computer system may be distributed over a computing cluster or cloud computing platform. Also, while reference is made to a conventional computer architecture hereinbelow, it will be clear to the skilled person that this is not necessarily the case in all embodiments. For example, the computer system may comprise, or even consist of, dedicated hardware for implementing the processing functions described hereinbelow, e.g. comprising an application specific integrated circuit and/or field-programmable gate array hardware. It will also be understood that, where reference is made to a single computer system, different functions of this computer system may be assigned to dedicated computer systems, e.g. which are able to exchange data.

The computer system 122 may comprise at least one processor 123. The processor may be operably connected to the hardware interface, such as to allow the gradient coils, the sensory stimulus source and the RF transceiver to be controlled by the processor and/or to enable processing of data received by the RF transceiver by the processor.

The computer system 122 may comprise a user interface 124, e.g. for interacting with an operator operating the MRI system. The processor may likewise be connected to the user interface, e.g. such that a user can select actions to perform by the MRI system, parameters to apply, review data indicative of the operation of the system and/or images as acquired by the system (or images derived therefrom). The user interface may comprise a display, a keyboard, a pointer device, a touch interface, and/or other such components as are ubiquitous in human-computer interaction systems. The user interface may be adapted to render images, e.g. via the display.

The computer system 122 may comprise a memory 125. The memory may comprise or consist of different types of memory, all of which are (directly or indirectly) accessible to the processor. This may for example include a random access memory, a cache memory, non-volatile memory (such as flash memory), hard drives, solid state drives, and/or other storage devices. The memory may comprise a non-transitory computer-readable medium.

The memory may contain machine-executable instructions to enable the processor to perform various data processing tasks and/or to control other components of the MRI system. The machine-executable instructions may enable the processor to perform (e.g. at least part of) a method in accordance with embodiments of the present invention.

A method in accordance with embodiments of the present invention may be embodied in a source program, an executable program (e.g. a bytecode or intermediate language code, an object code and/or a machine code), a computer-interpretable script and/or any other entity comprising a set of instructions that can be directly or indirectly (e.g. by means of facilitating programs or codes, such as interpreters, just-in-time compilers, compilers, etc., and/or supporting code libraries, frameworks, an operating system, etc.) executed by a computer system, e.g. by the processor of the computer system 122. It is pointed out that embodiments are not limited to computer code implementations, e.g. the method may be performed (completely or in part) by dedicated hardware specifically designed for the intended method, e.g. in an ASIC, and/or by a hardware configuration that can be loaded in a programmable hardware construct, e.g. an FPGA.

The memory 125 may also contain a representation of pulse sequence commands, which enable the processor to control the MRI system in a corresponding manner, e.g. by controlling the gradient coils and RF transceiver in accordance with these commands. The memory may also contain magnetic resonance imaging data acquired by controlling the MRI system with the pulse sequence commands.

The computer system 122 may also be adapted to reconstruct a tomographic image from MRI data acquired from the RF transceiver. Methods for controlling the MRI system using a suitable pulse sequence and for reconstructing tomographic images from the signals collected by the RF transceiver in response are well-known in the art, and will not be discussed in detail in the present disclosure.

The computer system 122 may comprise, e.g. may implement via suitable software and/or hardware, a sleep onset detection module to detect and/or predict the onset of sleep of the imaged subject, as explained in detail in the description of a method in accordance with embodiments of the present invention hereinbelow.

Referring to Fig. 2, a method 10 for monitoring the wakefulness of an imaged subject during a magnetic resonance imaging examination in accordance with embodiments of the present invention is shown.

The method 10 comprises acquiring 11 a magnetic resonance image of the subject using a magnetic resonance imaging system, e.g. an MRI system 100 as described hereinabove. For example, acquiring the image(s) may comprise controlling the MRI system in accordance with a pulse sequence, and collecting signals by the RF transceiver.

As illustrated in Fig. 2, the acquisition 11 of the MRI image(s) may typically take some time (schematically indicated by the time arrow T). Depending on the type of imaging procedure and the data to be collected, this timespan can be considerable, e.g. ranging from a few minutes to tens of minutes, or even (for example) an hour or longer. Therefore, a risk exists that the subject being imaged falls asleep during the procedure, which can be problematic, e.g. as already explained in the background section hereinabove. For example, a 'resting-state' scan, e.g. to determine and/or diagnose neural connectivity in the brain, can require a timeseries of volumetric images taken over an extended period of time, e.g. longer than 25 minutes, in order to determine correlations (and/or anticorrelations) of neural activity between different brain regions with a sufficient statistical significance. During this imaging procedure (without limitation thereto), the patient typically needs to refrain from movement (to avoid or reduce movement artefacts) and is not engaged in any activity, e.g. a sensory or cognitive task (as would be the case for conventional fMRI paradigms). Clearly, this is particularly boring, and a risk exists for the patient falling asleep. The fact that this procedure can be performed with the eyes closed is furthermore a contributing factor to this risk. However, the risk of falling asleep is not limited to such resting state scans alone (even though the risk of falling asleep might be particularly high in that illustrative case).

The "MRI image" may refer to one or more images, e.g. to a plurality of images, e.g. to a plurality of volumetric (3D) images. The MRI image may comprise a volumetric image (or more accurately, the acquired data allows a volumetric image to be reconstructed therefrom). The MRI image may comprise a plurality of images, e.g. a plurality of volumetric images. This plurality of images may be substantially different, e.g. may correspond to different scanner settings and/or anatomical parts being images, or may be substantially the same except for the time of acquisition (or a combination of both), i.e. the MRI image may comprise a timeseries of (e.g. volumetric) images. The MRI image may comprise an MRI image (or images) of the subject's brain, e.g. for applications (such as diagnosis and/or treatment or intervention planning) in neurology and/or psychiatry (and/or neuro-research). However, embodiments of the present invention are not necessarily limited to applications in brain imaging.

Acquiring the MRI image(s) may also comprise processing the collected signals to reconstruct an image, e.g. a tomographic image or tomographic images. The reconstructed image may be a volumetric (3D) tomographic image, one or more planar (cross-sectional; 2D) images, or a combination thereof. It is known in the art to reconstruct the signals in substantially real-time, i.e. while still acquiring the timeseries (before completion of the series), even though the immediate reconstruction may be performed (yet not necessarily) at a lower resolution, over a smaller region (e.g. only one or a few slices) and/or at a lower quality than a final reconstruction. Such final reconstruction may, for example, be performed at a later time when the image or images have been collected in its/their entirety, e.g. such that a diagnosis can be based on all information contained in the complete collection of acquired images, and/or at the highest achievable (or practically desirable) reconstruction quality and resolution of the acquired image(s).

In view of the state of the art in the field of MRI, the present disclosure will not go into detail regarding suitable pulse sequences, image reconstruction methods and/or MRI analysis techniques (e.g. additional processing of MRI images, such as to derive perfusion maps, diffusion tensor images, fMRI brain activity maps, etc.). The skilled person is considered to be well acquainted with suitable methods known in the art. However, it is specifically noted that techniques are known in the art to perform MRI image reconstruction in (near) real-time, as well as to detect brain activity in (near) real-time on the basis of such images (e.g. via estimation of the BOLD response), which may be applied in a method in accordance with embodiments of the present invention (without limitation thereto), as will be explained further hereinbelow.

The method 10 comprises determining 13 a wakefulness measure based on at least a part I of the acquired 11 magnetic resonance image or images and/or on electroencephalography (EEG) data from the imaged subject.

The image acquisition may comprise a prolonged acquisition, for example due to the image comprising a time series of dynamic images, a large number of images of the same or related body parts (e.g. using different sequence settings), and/or an image that in itself takes a long time to acquire (e.g. due to the imaging of a large volume, such as a whole-body scan, and/or at a high resolution). Therefore, the wakefulness measure may be determined on the basis of the partial acquisition, e.g. based on one or a few images in a series and/or on a partial reconstruction, e.g. in which only a part of a larger volume being imaged is reconstructed (e.g. one or a few slices of a larger image). In other words, the wakefulness measure may be determined during the acquisition of the image or images on the basis of the image(s) (or a partial image) that have already been acquired at the time that the wakefulness measure is being determined. After the wakefulness measure is determined, the acquisition of the image(s) may thus continue.

For example, the wakefulness measure may take into account brain activity in a predetermined region of interest, which is involved in the management of sleeping and/or waking states of the brain. This brain activity may for example be determined using an fMRI technique applied to the partially acquired image(s) or time series.

It is to be noted that the images being acquired 11 are not necessarily limited to fMRI data, e.g. BOLD-signal sensitive images. Nonetheless, the images being acquired may, as a part thereof, comprise fMRI data (i.e. brain scan images suitable to evaluate brain activity and/or regional neural connectivity). For example, the image or images being acquired may comprise a primary image or sequence of images of interest and a secondary image or sequence of images specifically intended to determine the wakefulness measure, in which this first and second image (sequence) can, for example, be obtained in an alternating fashion. In other words, the image or images of primary interest (i.e. being the intended purpose of the MRI examination) can be collected intermittently; interspersed with the collection of MRI image(s) or signal(s) specifically intended to determine the wakefulness measure.

It is also to be noted that, if the primary sequence is not suitable for determining this measure of wakefulness, including additional images or signals into the acquisition procedure, specifically for this purpose of determining the measure of wakefulness, does not necessarily create an excessive overhead. Only sufficient data needs to be collected to sample the region of interest, with a sufficient, but possibly quite low, spatial resolution (and/or temporal resolution, e.g. if the wakefulness measure is determined repeatedly during the procedure). Since this secondary information may be used to determine the measure of wakefulness automatically, the image quality can be substantially lower (not necessarily though) than the quality that would be required for interpretation by a human observer.

This determining 13 of the wakefulness measure based on at least a part of the acquired 11 magnetic resonance image or images may comprise evaluating a contrast image, e.g. by comparing (e.g. differencing) an image (or images) captured with the subject in a known state, e.g. fully awake, to a current image (or images). Alternatively, a template image may be used as reference that is not necessarily obtained from the same subject, e.g. using a reference image obtained from statistical analysis of multiple subjects, e.g. a reference population. Information from a region of interest (e.g. one or more pixels/voxels) may also be extracted directly from a single image (or images) and compared to a reference value or processed to determine an indicator value, e.g. without relying on a reference image.

As an illustrative example, the ventrolateral preoptic nucleus (or a part thereof) of the hypothalamus may be monitored, e.g. the intermediate nucleus\ventrolateral preoptic nucleus (IN\VLPO), and/or adjacent and functionally related structures, e.g. the median preoptic nucleus (MnPO). It is an advantage of monitoring this region(s) of interest that a transition from waking to the initial stage of sleep, e.g. a transition into slow-wave sleep, can be detected efficiently. It can be presumed (without limitation) that the onset of sleep is triggered by the inhibition of systems for maintaining a waking state, in which this inhibition is instigated by neurons in the VLPO that are specifically activated in sleeping state(s). At the onset of sleep, these VLPO neurons may be activated by various mechanisms, e.g. primarily but not necessarily solely by the suprachiasmatic nucleus (circadian clock) and/or hypnogenic factors. It may be important to note that, while various regions or structures may be involved in sleep (e.g. may have a different characteristic activity pattern in a sleeping state as compared to a waking state), the VLPO may act as an initiator and therefore monitoring the VLPO (and/or MnPO) may allow the onset of sleep to be detected and even predicted very efficiently.

Thus, determining 13 the wakefulness measure may comprise extracting one or more image values from the MRI image(s), in which these value(s) correspond(s) to the VLPO, e.g. directly or by computing a suitable contrast value from the image values. For example, a method as known in fMRI processing may be applied to derive a value indicative of a BOLD signal (or BOLD contrast) in the region of interest. The neuronal activity in a region of interest that is related to the sleep/waking states, such as the VLPO and/or MnPO, can be effectively monitored by (f)MRI imaging, e.g. using an MRI sequence that is sensitive to transverse relaxation (T2*). For example, a transverse relaxation map obtained after processing the observed RF signals may be compared to a reference T2* map, which may be obtained from the same subject in a known state (e.g. fully awake, for example obtained early on in the image acquisition procedure, when alertness can be easily confirmed or assumed by an operator) or synthetically constructed (e.g. by combining images obtained from a reference population sample). A blood-oxygen-level dependent (BOLD) response of the region (or regions) of interest (which may be an increase or a decrease with respect to a reference) can be determined using the results of such comparison. However, it is to be noted that this is only an example, and alternative approaches can easily be envisioned by the skilled person to determine an equivalent indication value of activity in the region or regions of interest.

For example, the posterior hypothalamus region may show wake-promoting activity, e.g. may deactivate when sleep onset occurs, and/or the anterior hypothalamus may show sleep-promoting activity, e.g. may activate when sleep onset occurs. Thus, such behavior in these regions (either one in isolation or in combination; not necessarily limited to these specific regions) may be tested using an approach as discussed hereinabove and used in determining the wakefulness measure.

Embodiments may also apply a functional connectivity method, in which the activity in different regions of interest are correlated to determine a value indicative of wakefulness (or an element used to determine such measure by further calculations). A functional connectivity measure may be determined to indicate wakefulness, for example by evaluating a functional connectivity between the VLPO and the MnPO (e.g. emphasizing the aforementioned anticorrelation between sleep-promoting and wakefulness-promoting parts of the hypothalamus), between VLPO and/or MnPO and other sleep-related regions (e.g. the upper brainstem, the ventral anterior insula, the pregenual anterior cingulate, etc.), and/or combinations thereof.

In other words, determining the wakefulness measure may comprise a conventional fMRI processing (e.g. using a general linear model with an underlying model of time-dependent changes in observed image intensity to indicate changes in wakefulness) and/or a resting-state functional connectivity fMRI analysis (rs-fcMRI).

Examples of differences in neural activity, as can be detected by MRI (e.g. via the BOLD effect), that are indicative of wake and sleep states are known and documented in the art. For example, Tagliazucchi and Laufs, "Decoding Wakefulness Levels from Typical fMRI Resting-State Data Reveals Reliable Drifts between Wakefulness and Sleep" in Neuron 82(3), pp. 695-708, describes an analysis of resting-state fMRI data sets. The transition from wake to sleep was characterized by substantial changes in the neuronal activity .The spatial and functional extent of these changes can therefore be used to detect this state transition. A further example of documented changes in neural activity due to wakefulness/sleep dynamics, as can be detected by MRI, can be found in Stevner et al, "Discovery of key whole-brain transitions and dynamics during human wakefulness and non-REM sleep" in Nature Communications 10, article no. 1035.

To determine the wakefulness measure, only a few images, one image, or even a partial image may suffice. Unlike a conventional processing method of (e.g.) fMRI data, the processing of MRI data to determine the wakefulness measure can be performed very rapidly, e.g. in near real-time, for example due to the fact that the wakefulness measure can be determined on the basis of only a specific predetermined region or only a few regions of interest and can be based on only one or a few images. This does not mean that more images cannot be taken into account. For example, the images on the basis of which the wakefulness measure is determined may comprise all (or many; or most) of the images in a timeseries that have been collected up to the point of calculating the wakefulness measure, and, for example, a time regressor may be applied and/or a time smoothing (e.g. exponential smoothing) may be applied (without limitation to these examples) to obtain a wakefulness measure indicative of a current (i.e. near instantaneous) state of the imaged subject.

Determining 13 the wakefulness measure is not necessarily based on, or not necessarily solely based on, magnetic resonance image or images of the brain. For example, determining 13 the wakefulness measure may comprise determining one or more cardiorespiratory parameters based on the (at least part of the) acquired magnetic resonance images. These cardiorespiratory parameters may particularly be dynamic cardiorespiratory parameters, e.g. parameters indicative of a present (or at least very recent) activity of the cardiorespiratory system, such as a respiratory rate, a heart rate, the variability of respiratory and/or heart rate, a (virtual; i.e. determined from MRI data) photoplethysmogram (PPG), etc. It will be understood that such cardiorespiratory parameters are not necessarily based on brain imaging (but can be). It is known that the onset of sleep can be detected by monitoring such cardiorespiratory parameters, e.g. by detecting a slowing of heart and/or respiratory rate and/or a change in variability thereof. Sleep onset may be associated with a shift toward increased parasympathetic modulation, which can be detected from the cardiorespiratory parameter(s). It is also noted that the image(s) being acquired are not necessarily limited to the type of image, or the imaged anatomical parts, from which the cardiorespiratory parameter(s) are determined, e.g. short acquisitions may be inserted in an extended MRI sequence to detect the parameters of interest at one or more times of interest interspersed in between signal acquisitions for reconstructing the actual images of interest (i.e. relating to the purpose of the examination).

It is an advantage of embodiments of the present invention that no additional equipment, such as sensors, is needed to detect a patient falling asleep during an MRI imaging session. However, this does not preclude embodiments comprising the use of such sensor equipment, e.g. to improve the accuracy of the wakefulness measure or to avoid changes in the imaging protocol.

Determining 13 the wakefulness measure may take, e.g. in addition to information gathered from the acquired image(s), sensor data S into account, and the method may accordingly comprise the collection 12 of such sensor data S to be used as (e.g. further) input in determining 13 the wakefulness measure.

For example, a sensor or sensors may be used to monitor one or more physiological parameters that could be indicative of a sleep and/or waking state, and/or could correlate with (in the general sense; e.g. the sensor signal being informative for determining) a transition from a waking state to a sleeping state (or, obviously, vice versa). Such sensor may be adapted for acquiring (e.g. preferably repeatedly during the examination) an electroencephalogram (EEG), an electrocardiogram (ECG), a photoplethysmogram (PPG), a cardioballistogram (CBG), and/or an electromyogram (EMG; e.g. of muscles directly or indirectly involved in breathing motion), and/or may be adapted to monitor the breathing and/or cardiac activity (e.g. breathing rate; heartrate) using a radar (or lidar) system, and/or an optical camera system and/or an infrared vital signs monitoring camera system. While reference is made to a 'sensor' or sensors, it will be understood that this should not be construed narrowly, e.g. the 'sensor' may include equipment for processing a signal input from a sensor (in the narrow sense) to a more informative output (e.g. estimating a respiration cycle phase and/or respiration rate from a camera image).

The sensor data may be collected 12 substantially simultaneously with collecting the part I of the image data used for determining the wakefulness measure, or with a known relationship between the timing of the sensor data capture and the image acquisition sequence.

Collecting 12 the sensor data may comprise collecting cardiorespiratory data (referring to data that is indicative of a, e.g. vital, parameter or parameters of the cardiovascular and/or respiratory system) from the imaged subject using one or more sensors, e.g. a pulse oximetry device (e.g. a PPG, photoplethysmography, device), a cardiac monitoring device, a respiration monitoring device, and/or combinations thereof. Examples of cardiac monitoring devices include a heart rate monitor, a Holter monitor, an electrocardiography (ECG) device, and/or a ballistocardiography (BCG) device, without limitation thereto. Examples of respiration monitoring include a spirometry device, a camera observation system to track breathing motion, a radar monitoring system to track breathing motion, and/or similar systems. The sensor system may be adapted to monitor multiple parameters at once, and is not necessarily limited to cardiorespiratory parameters. For example, the sensor system may comprise a camera system for monitoring (vital) signs, which may include a heart rate and/or a respiration rate, but also information about generic movements and/or behavioral information (e.g. detecting changes in behavior).

Collecting 12 the sensor data may also comprise tracking motion and/or dilation of the eye pupil(s) of the subject, e.g. using an MRI scanner-compatible eye tracking method as known in the art. For example, a lack of variability of the eye motion and/or a decreased size and/or a decreased variability of pupil size may indicate an increased risk of falling asleep, which may be taken into account in determining the wakefulness measure.

Collecting 12 the sensor data may comprise collecting electroencephalography (EEG) data from the imaged subject, i.e. the sensor system may comprise an EEG system. It is known in the art that sleep states can be characterized by their substantially different electroencephalography traces (and/or frequency bands) when compared to waking states. It is known that the onset of sleep, e.g. a transition of diminishing alertness and wakefulness, can be efficiently detected in EEG data. By combining EEG data and MRI data, such as 'virtual' PPG data and/or BOLD effect changes in the VLPO or similar brain regions that are indicative of the sleep/wake cycle, a very efficient approach to detecting the onset of sleep, and even predicting the transition with some margin (in time) can be provided. For example, the application of concurrent EEG and fMRI has been used extensively to study sleep. EEG offers an excellent temporal resolution, but poor spatial localization of phenomena in the brain, such that fMRI data forms an ideal complement.

Even though the combination of EEG and MRI data to detect the onset of sleep in a robust and reliable manner may be preferable, it is to be noted that embodiments in which the wakefulness measure is determined 13 on the basis of EEG data without further relying on the (at least part I of the acquired) magnetic resonance image or images is not necessarily excluded. Likewise, embodiments that rely on the imaging (MRI) data, without necessarily requiring EEG data, are not excluded either.

For example, it is known in the art that even a single electrode (e.g. in combination with a reference potential electrode) may provide an EEG signal that is sufficient to detect and even predict the onset of sleep. For example, the electrode may be positioned at a frontal position (e.g. held in place by a headband). Zhang et al, "Automatic sleep onset detection using single EEG sensor" in Annual International Conference of the IEEE Engineering in Medicine and Biology Society (EMBC) 2014, pp. 2265-2268 (doi: 10.1109/EMBC.2014.6944071), describe such method for detecting and predicting sleep onset based on a single frontal electrode EEG signal. However, many alternative approaches are publicly available, and embodiments are therefore not considered to be limited to this illustrative approach.

The wakefulness measure is not necessarily a scalar, e.g. may comprise a vector of different values. The wakefulness measure can be (or comprise) a discrete value and/or a continuous (real) value. Preferably, the determined 13 wakefulness measure comprises or consists of a prediction of the subject falling asleep, e.g. an estimate of a time to onset of sleep and/or an indicator expressing a likelihood of falling asleep within a predetermined time period (e.g. starting from the time on which the data was collected on which the measure is based). The indicator may be a Boolean value (e.g. expressing a likelihood above a predetermined threshold), but may also be indicative of the estimated probability or likelihood, e.g. a real value proportional to the probability.

While embodiments of the present invention do not necessarily rely on machine learning methods, it is noted that the wakefulness measure can be determined by a suitable machine learning method that receives the image data and/or EEG data, and/or image/signal features determined therefrom, as input and that is trained to respond with the wakefulness measure as output. It is noted that a suitable training set can easily be obtained by recording data and annotating this data, in hindsight, with relevant sleep information. Thus, the wakefulness measure, as trained by such approach, can include a prediction of time until onset of sleep, an indicator variable to indicate that a sleep cycle will initiate within a predetermined time period, or similar measures. Suitable machine learning techniques may include: neural networks, deep learning networks, classification or regression trees (or derivatives thereof, e.g. regression/classification forests), support vector machines and the like.

It is however to be noted that determining the wakefulness measure does not necessarily rely on machine learning techniques. For example, methods are known in the art to detect the onset of sleep on the basis of a deterministic algorithm (e.g. designed and readily comprehensible by a human) applied to the MRI imaging data (e.g. thresholding a BOLD contrast observed in the VLPO) and/or the EEG data (e.g. thresholding a power ratio of different frequency bands, e.g. expressing alpha vs. theta wave power). The MRI and/or EEG data, and possibly further sensor data, may, for example, be processed using techniques as known in the field of polysomnography as the basis for (or an element in) determining the wakefulness measure.

As already mentioned, the MRI and/or EEG data may be supplemented by further sensor information. Using the MRI and/or EEG, and possibly further sensor signals, the wakefulness measure can be determined such as to detect the onset of sleep as well as to accurately predict the sleep onset, e.g. within the next 30 seconds. Different combinations of data can also be used to achieve sufficient accuracy in predicting the onset of sleep, such as MRI-based imaging in combination with a PPG or cardiorespiratory sensor signal. It is to be noted that cardiorespiratory parameters and/or PPG data can also be derived from MRI image data, such that good predictive power can be achieved by relying on solely MRI data (e.g. BOLD monitoring of the VLPO in combination with virtual PPG, without limitation to this example).

The method 10 further comprises generating 14 a signal, e.g. an alert signal, when the wakefulness measure indicates that the subject has fallen asleep (or woken up, e.g. if the aim of the examination is a sleep study) and/or is likely to fall asleep (or to wake up). As already mentioned, preferably, the wakefulness measure may consist of or comprise predictive information, such as a probability of the subject falling asleep (e.g. withing a predetermined time period), an estimated time until sleep onset and/or an indicator of a high risk of falling asleep. Thus, the signal may be generated 14 when it is considered likely that the subject being imaged will fall asleep (or will wake up), e.g. within the next 30 seconds (without limitation to this illustrative timeframe).

The signal may be generated before completion of the acquisition 11 of the MRI image(s), e.g. such that action can be taken by an operator during the further acquisition of the MRI image(s). In other words, the signal is generated during the acquisition 11 and/or before removing the subject from the magnetic resonance imaging system.

The signal can be considered as an (conditional) output of the method, but is not necessarily an 'output' of a system or device executing the method, e.g. the signal may be entirely internal to the system, e.g. activating a subroutine to extend, pause, terminate, and/or restart the MRI examination. It will also be clear that the signal may comprise one signal or a plurality of separate signals, which may be of a similar or different nature (e.g. a value stored in a memory, a message displayed on a screen, an auditory cue, a computer network transmission, etc.).

The method in accordance with embodiments of the present invention thus provides a (near) real-time response to a subject falling asleep during MRI imaging, and preferably before the onset of sleep (by a predictive detection).

The generated 14 signal may alert an operator when the patient has fallen asleep or is about to fall asleep, such that the operator can take a corrective or responsive action, such as waking the patient up, restarting the imaging procedure, extending the duration of the imaging procedure (e.g. adding images to a timeseries of images being collected) and/or related actions. For example, the alert signal may be provided to an operator via the user interface 124 of the computer system 122. Thus, the operator's attention is drawn to the fact that the subject may fall asleep or may have fallen asleep. The operator can then take action, before the image acquisition has been completely terminated, to alleviate this problem.

Furthermore, the operator may be warned by such signal (or a component thereof) before an automatic action is taken, e.g. to wake the subject up or increase the subject's alertness. Thus, the operator can override such an automatic action when it is deemed unnecessary or it is deemed more appropriate to personally intervene.

The generated 14 signal is not necessarily limited to merely providing an informative prompt to the operator. In a method in accordance with embodiments of the present invention, the signal may comprise (additionally or alternatively to informing an operator) a cue to the subject to refocus attention and increase alertness. The generated 14 signal may thus (directly and automatically) wake the subject up and/or alert the subject to prevent the onset of sleep.

For example, the sensory stimulus source 120 of the MRI system may be used for providing such feedback/alert to the subject, e.g. as an audio and/or video cue (without necessarily being limited thereto). The sensory stimulus source may comprise an audio system (e.g. headphones) and/or a video system (e.g. a display). The sensory stimulus source may comprise a light, such as a dedicated light source to provide a stimulus to wake the patient up or to prevent the patient from falling asleep, or a controller of ambient lighting in the MRI room.

Examples include an increasing volume of a sound, e.g. background music, a change of music presented to the subject, e.g. switching to a more energetic type of music, a bright and/or flashing light (e.g. via ambient lighting, a dedicated light source, such as a spot light, and/or a light source integrated in a visual presentation or video delivery system), an increased intensity of a display that the subject can view during the exam, etc. In other words, the stimulus presented to the subject may be specifically provided to wake/invigorate the subject, but may also consist of or comprise a change in content or control settings of an audio/visual stream (and/or of ambient lighting, and/or of one or more dedicated light or sound sources) being presented to the subject during the (e.g. entire) MRI session.

Examples of alternative (or additional) sensory stimulus sources include: an olfactory stimulator (e.g. by releasing a pungent compound in or near the nose), a tactile stimulator (e.g. a haptic feedback device), and/or a thermal stimulator (e.g. a localized heat source or sink, and/or an ambient heating/cooling device).

Additionally and/or alternatively, the MRI system may be controlled to wake the subject up, or increase his/her alertness, by a movement of the subject. Such movement may be delivered via an actuated patient couch 112 of the MRI system, and/or via a separate actuator (e.g. for delivering a more subtle movement that is less likely to interfere with the imaging protocol). Examples of the latter may include an actuator configured to move, e.g. shake, a hand or foot of the subject, or even less intrusive, such as applying a vibration to the skin of a body part. Another form of tactile feedback signal is also possible (e.g. using a haptic feedback device).

Another example includes purposefully controlling the MRI system to cause a distinct sound or noise, e.g. as typically occurs due to gradient switching. By temporarily pausing the imaging sequence and executing a sequence that is specifically intended to cause a perceptual change in sound, the attention of the subject can be caught and the onset of sleep may be prevented.

Other possible examples include the delivery of a stimulus via electrodes (e.g. applied on the skin of the subject). For example, when the wakefulness measure is determined based on (or partly based on) EEG signals, the EEG electrodes can also be used to deliver a mild current, causing a tingling sensation. This however does not exclude embodiments in which further electrodes, e.g. in addition to those used for EEG observation, are included specifically for the purpose of delivering a stimulus. As another example, a chemical stimulus may be used, e.g. by releasing a compound that can be tasted or smelled by the subject or that creates a sensation on the skin.

The signal may alternatively or additionally comprise a control signal to control a thermal unit, e.g. a heater and/or cooler to increase or reduce a temperature to which the subject is exposed (e.g. a room temperature, without limitation thereto). The signal may alternatively or additionally comprise a control signal to control an air flow to which the subject is exposed, e.g. a fan. As already mentioned, a thermal signal may also be delivered to the patient in a local manner, e.g. by a heat conductive channel locally touching the skin and connected to a heater or cooler (e.g. a Peltier element and/or ohmic heater).

A combination of several of such modes to deliver an alert signal to the subject may be used to wake the subject up or to increase his/her alertness, e.g. the signal may comprise a multi-sensory signal.

A sensory or multisensory signal provided to the subject may be delivered in a stepwise and/or gradual manner (i.e. as opposed to switching only between an 'off' and 'on' state). For example, the intensity of a sound, light (or image or video), a movement, a vibration, a temperature controller, an air flow, etc. may be gradually increased (or, where appropriate, decreased) to avoid startling the subject. Likewise, a parameter or parameters (i.e. not necessarily limited to an intensity) may be continuously (or in small steps) varied to create a gentle stimulus. Examples of parameters other than intensity-related parameters include a frequency, a spectral property (e.g. color hue, color saturation), a temperature, and a position where a stimulus is applied (this list not exhaustive). By providing a signal (stimulus/stimuli) to the subject in a gradual and gentle way, a startle response with consequently potential undesirable movement can be prevented, while the stimulus can be increased up to a perceptual level that is sufficient to evoke a conscious experience to increase alertness. This may be particularly advantageous in combination with a predictive wakefulness measure, since a gradual increase of the stimulus may take some (limited) time to reach a sufficient level, e.g. to pass a perceptual threshold.

For MRI examinations in which stimuli are provided to the subject as part of the imaging protocol, e.g. an fMRI examination, the sensory or multisensory signal that is provided to the subject to prevent the onset of a sleep epoch (or wake the subject up) can be selected such as to not intervene with the imaging protocol. In other words, the imaging protocol may involve the intentional activation of a specific brain region(s) due to sensory stimuli (and/or activated by the subject carrying out a task as instructed when presented by such stimulus), and the sensory or multisensory signal may be selected such as to avoid activation of the same brain region(s). For example, the imaging protocol may use visual stimuli, while the signal to prevent sleep or promote a waking state can use an audio signal (without limitation to this illustrative example).

Generating 14 the signal may also take past actions into account. For example, the intensity of an applied stimulus to the subject to promote alertness and/or to prevent sleep may be automatically increased if previously similar actions were taken. Thus, if a subject appears to be very prone to falling asleep during an examination, the intensity of the applied stimuli may be increased (or a different relevant parameter may be changed; or a different type of stimulus may be applied).

The generated signal may comprise an instruction to the computer system 122 controlling the MRI system 100 to (e.g. automatically) extend a time series of images being acquired 11, to repeat the acquisition 11 of the image (or part of the images being acquired), to terminate the acquisition and/or to pause the acquisition, for example to allow an operator to wake the subject up or to ensure that the subject remains awake.

For example, the method may comprise monitoring the acquisition time and/or the number of images that correspond to a waking state (or, if more appropriate for the intended purpose, a sleeping state) of the subject, as indicated by the determined wakefulness measure (which preferably is repeatedly, e.g. frequently, updated). Thus, this monitoring may comprise calculating the time or the number of images (e.g. frames of a timeseries of images, such as an fMRI timeseries) during which the subject was awake. The MRI acquisition may be extended, e.g. by the generated signal providing a suitable instruction to the computer system 122, to ensure that a predetermined number of images or a predetermined time of imaging the subject in the waking state (or sleeping state, if preferred in view of the intended purpose) is achieved. By monitoring the wake/sleep state of the subject and extending the data acquisition as needed, an autonomous approach (or semi-autonomous in case an operator is requested to confirm such extension) to quality assurance is provided, e.g. the procedure is not terminated until a sufficient number of wakeful scanning minutes or a sufficient number of wakeful images have been collected, such that rescheduling for a repeated MRI examination caused by poor data quality can be avoided (or is needed less frequently). Variations on this approach can also be envisioned, such as, instead or in addition to ensuring a total number of wakeful minutes/frames, ensuring at least one (or another predetermined number of) continuous time block(s) of at least a predetermined length in time (or comprising a predetermined minimum of collected images) in which the subject is in the waking state (or sleeping state, if preferable). In other words, the image acquisition protocol may be automatically (or semi-automatically) repeated and/or extended until a predetermined period of scanning has been reached (or number of captured images) without detected sleep onset epochs.

The generated signal may comprise annotation information (e.g. metadata) that is stored along with the MRI image data being acquired, such that the wakefulness measure, e.g. a Boolean or non-Boolean value to indicate a waking vs. sleeping (or reduced wakefulness) state, can be taken into account when processing the MRI data after completion of the examination. For example, each acquired image (e.g. which may be part of a time series) may be marked with an indicator variable to indicate if the subject was awake or asleep at the capture time, or with a non-Boolean variable, e.g. a probability estimate of the subject being asleep.

Alternatively or additionally, the annotation information may also indicate when a sensory stimulus was provided to the subject to wake the subject up and/or to alert the subject to prevent the onset of sleep (e.g. by annotating the corresponding frame in a timeseries with a flag and/or a timestamp that the stimulus was applied). This allows the effects in the brain of the stimuli that were provided to wake the subject up and/or to promote wakefulness to be separated in an analysis of the data from the effects of an fMRI task and/or of other stimuli that were provided as part of an fMRI protocol.

For example, the wakefulness measure, or a value derived therefrom, may be used as a covariate in performing an fMRI analysis of the collected imaging data, e.g. as a covariate in a general linear model applied to the timeseries. Alternatively or additionally, a part or parts of the acquired image or images for which the annotation information indicates a reduced wakefulness or sleeping state may be rejected (e.g. discarded) when analyzing (automatically processing and/or manually evaluating) the results. It is noted that the effect of sleep onset or sleep epochs can also be removed (or compensated) in an analysis of the collected data, using the annotation information, if the fMRI data is not collected in accordance with a conventional task-based paradigm (e.g. a blocked or event-based paradigm). For example, the sleeping/waking annotation and/or stimulation annotation information can be separated from resting-state MRI (or EEG/MRI) examination data by a time-locked analysis.

The wakefulness measure is not necessarily a scalar value, e.g. may comprise a plurality of different indicators and/or values, and the generated 14 signal may comprise a plurality of different signals that can be generated in response to different conditions based on different components of such multi-dimensional wakefulness measure. For example, the wakefulness measure may indicate a condition of the subject being asleep differently as a condition in which the onset of sleep is predicted but has not yet occurred. Thus, different responsive actions can be taken accordingly, e.g. to merely promote alertness of the subject while still awake and to wake the subject up when detected as already being asleep. The type of response may differ substantially, or merely in intensity (or other parameter), e.g. by applying a subtle movement to the patient while still awake but at risk of falling asleep and a less subtle movement when confirmed to be asleep.

Generating 14 the signal may comprise generating a plurality of signals, e.g. a combination of two of more examples given hereinabove. The term 'signal' may be interpreted broadly, and the signal, or components of the signal, may include digital data, e.g. stored in a memory or transmitted via a transient or non-transient data carrier, information presented in a user interface, physical signals delivered via the stimulus system, and/or any other useful form of data representation that can be interpreted by a computer system (the same or a different computer system as the system where the signal is generated) and/or human (e.g. the operator or the subject), as would be evident from the type of information being carried by the signal.

The method may also comprise repeatedly 15 determining 13 the wakefulness measure (based on current data, i.e. each instance of determining the wakefulness measure being based on data representative of a present state of the subject at/near the moment of said determining). Likewise, the signal may be generated 14 based on each instance of the determined 13 wakefulness measure, e.g. such as to monitor the wakefulness of the imaged subject repeatedly throughout the image acquisition 11 and taking action (in the form of the generated 14 signal and its effects) when needed, i.e. when the subject is falling asleep or is deemed to be at a high risk of falling asleep. The wakefulness measure may thus be (e.g. repeatedly) updated to indicate a present (current; substantially actual) state or degree of wakefulness, and, hence, the signal, such as an alert, can be generated 14 at different points in time during the MRI examination when the risk of the subject falling asleep is estimated to be high (e.g. the estimate being above a predetermined threshold), when the subject has fallen asleep and/or when occurrence of sleep within a predetermined timeframe, e.g. less than a minute, e.g. less than 30 seconds, is predicted.

Thus, the method may comprise repeatedly 15 determining 13 the wakefulness measure to monitor the wakefulness of the subject throughout the acquisition 11 and may comprise generating 14 said signal whenever the most recently determined wakefulness measure indicates that the subject has fallen asleep, has woken up and/or is predicted to transition from a wake to a sleep state and/or vice versa.

In a second aspect, the present invention relates to a computer program product comprising machine executable instructions for execution by a processor, wherein execution of the machine executable instructions causes the processor to perform a method in accordance with embodiments of the first aspect of the present invention. For example, the processor may be (or may be comprised in) the illustrative computer system 122 of the illustrative MRI system 100 (see e.g. FIG 1 and the description hereinabove).

In a third aspect, the present invention relates to a computer system for monitoring the wakefulness of an imaged subject during a magnetic resonance imaging examination, for example a computer system 122 as discussed hereinabove.

The computer system comprises an input 131 for receiving, in substantially real-time, at least one magnetic resonance image of the subject acquired using a magnetic resonance imaging system 100, e.g. a timeseries of functional magnetic resonance images of the brain of the subject (without limitation thereto), and/or electroencephalography data obtained from the imaged subject during said magnetic resonance imaging. For example, the input may be comprised in the hardware interface 121 of the computer system 122. In case where the computer system does not receive (substantially real-time) MRI images, but receives EEG data as input (possible in combination with further sensor signals), the input may be configured to receive timing information regarding the concomitantly executed MRI imaging as input, e.g. a synchronization trigger signal(s).

The computer system comprises an output 132 for providing a generated signal to the MRI system and/or an operator of the MRI system and/or to the subject being imaged. The output 132 may be comprised in the hardware interface 121, in a user interface 124, in a memory 125, or in a combination thereof.

The computer system comprises a processor 123 for determining a wakefulness measure based on at least a part I of the acquired image or images and/or on said electroencephalography data, and for generating the signal if the determined wakefulness measure indicates that the subject has fallen asleep, has woken up and/or is predicted to transition from a wake to a sleep state and/or vice versa.

The processor is adapted to generate this signal during the image acquisition and/or before removing the subject from the magnetic resonance imaging system, e.g. before a stream of the data being received via the input has terminated. For example, the processor 123 may be adapted to generate the at least one signal during the acquisition of a timeseries of images (e.g. fMRI images).

In a fourth aspect, the present invention relates to a magnetic resonance imaging system comprising the computer system in accordance with embodiments of the present invention, e.g. a magnetic resonance imaging system 100 as discussed hereinabove.

The computer system and/or magnetic resonance imaging system in accordance with embodiments may be, particularly, adapted to perform a method in accordance with embodiments of the first aspect of the present invention, such that other features, or details of the features described hereinabove, of the MRI system and/or computer system in accordance with embodiments of the present invention shall be clear in view of the description provided further hereinabove relating to a method in accordance with embodiments of the present invention.

## Claims

1. A method (10) for monitoring the wakefulness of an imaged subject during a magnetic resonance imaging examination, the method comprising:
- acquiring (11) at least one magnetic resonance image of the subject using a magnetic resonance imaging system (100),
- determining (13) a wakefulness measure based on at least a part (I) of the acquired image or images and/or on electroencephalography data obtained from the imaged subject during said acquiring, and
- generating (14) at least one signal if the determined wakefulness measure indicates that the subject has fallen asleep, has woken up and/or is predicted to transition from a wake to a sleep state and/or vice versa, wherein said signal is generated during said acquisition (11) and/or before removing the subject from the magnetic resonance imaging system.

2. The method of claim 1, wherein said determined (13) wakefulness measure comprises or consists of a prediction of the subject falling asleep within a predetermined period of time.

3. The method of claim 1 or claim 2, wherein said at least one magnetic resonance image comprises a timeseries of magnetic resonance images, and wherein said wakefulness measure is determined when some, but not all, of the images in said timeseries have been acquired (11).

4. The method of any of the previous claims, wherein said wakefulness measure is determined (13) by taking brain activity into account in at least one predetermined region of interest of the brain that is involved in the management of sleeping and/or waking states of the brain, wherein said brain activity is detected by a blood oxygenation level dependent effect in said region of interest in said part (I) of the acquired image or images.

5. The method of claim 4, wherein said at least one predetermined region of interest comprises or consists of the ventrolateral preoptic nucleus and/or the median preoptic nucleus of the hypothalamus.

6. The method of any of the previous claims, wherein said wakefulness measure is determined (13) by taking at least one cardiorespiratory parameter into account that is determined based on said part (I) of the acquired image or images.

7. The method of any of the previous claims, in which acquiring (11) the at least one magnetic resonance image comprises acquiring a primary image or images, in which said acquisition (11) switches from acquiring a primary image or images of the subject for a diagnostic or other medical purpose to acquiring a secondary image or images forming said part (I) for determining (13) the wakefulness measure and back to acquiring said primary image or images.

8. The method of any of the previous claims, in which said determining (13) of the wakefulness measure takes further sensor data (S) into account that are collected (12) during said acquiring (11) of the image or images, wherein said further sensor data comprises an electrocardiogram, a photoplethysmogram, a cardioballistogram, an electromyogram, an optical or infrared camera image or video, and/or radar or lidar data.

9. The method of any of the previous claims, in which generating (14) said signal comprises generating an alert for an operator of the magnetic resonance imaging system to inform the operator that the subject has fallen asleep or is predicted to fall asleep, and/or comprises an instruction to the magnetic resonance imaging system (100) to extend a time series of images being acquired (11), to repeat the acquisition of the image or images or part thereof, to terminate the acquisition and/or to pause the acquisition.

10. The method of any of the previous claims, in said generated (14) signal comprises a sensory stimulus cuing the subject to wake the subject up and/or to reduce the risk of the subject falling asleep.

11. The method of claim 10, in which said stimulus is delivered in a stepwise or continuously varying manner so as to draw the attention of the subject without startling the subj ect.

12. The method of any of the previous claims, said generated (14) signal comprising annotation information for storing along with the acquired (11) magnetic resonance images, such that an evolution of the wakefulness measure during the acquisition and/or a timing of cuing the subject with said sensory stimulus can be taken into account when analyzing said magnetic resonance images.

13. A computer program product comprising machine executable instructions for execution by a processor, wherein execution of the machine executable instructions causes the processor to perform a method in accordance with any of the previous claims.

14. A computer system (122) for monitoring the wakefulness of an imaged subject during a magnetic resonance imaging examination, the computer system comprising:
- an input (131) for receiving, in substantially real-time, at least one magnetic resonance image of the subject acquired using a magnetic resonance imaging system (100) and/or electroencephalography data obtained from the imaged subject during said magnetic resonance imaging,
- an output (132), and
- a processor (123) for determining a wakefulness measure based on at least a part (I) of the acquired image or images and/or on said electroencephalography data, and for generating at least one signal via said output if the determined wakefulness measure indicates that the subject has fallen asleep, has woken up and/or is predicted to transition from a wake to a sleep state and/or vice versa, wherein said processor is adapted to generate said signal during said image acquisition.

15. A magnetic resonance imaging system (100) comprising said computer system (122) of claim 14.
